# EUROPEAN PATENT APPLICATION

(11) **EP 2 656 865 A1**
(43) Date of publication of application: **30.10.2013**
(21) Application number: 12165401.6
(22) Date of filing: 24.04.2012
(51) Int. Cl.: A61M 5/20, A61M 5/32, A61M 5/24, A61M 5/31

(54) **Syringe carrier with needle shield and data storage device for use in a medical auto-injection device**

(71) Applicant: Sanofi-Aventis Deutschland GmbH, 65929 Frankfurt am Main (DE)
(72) Inventor: The designation of the inventor has not yet been filed

(57) **Abstract**

Described is a syringe carrier for use in a medical auto-injection device comprising a housing (200) adapted to contain a syringe (102) containing a medicament, a shield (202) telescopically coupled to the housing (200) and translatable relative to the housing (200) between a retracted position and an extended position, and a data storage device (400) including medicament data.

## Description

### Technical Field

The invention relates to a medicament cartridge.

### Background of the Invention

Administering an injection is a process which presents a number of risks and challenges for users and healthcare professionals, both mental and physical.

Injection devices typically fall into two categories - manual devices and autoinjectors. In a conventional manual device, a user must provide force to drive a medicament through a needle. This is typically done by some form of button / plunger that has to be continuously pressed during the injection. There are numerous disadvantages for the user from this approach. For example, if the user stops pressing the button / plunger, the injection will stop and may not deliver an intended dose to a patient. Further, the force required to push the button/plunger may be too high for the user (e.g., if the user is elderly). And, aligning the injection device, administering the injection and keeping the injection device still during the injection may require dexterity which some patients (e.g., elderly patients, children, arthritic patients, etc.) may not have.

Autoinjector devices aim to make self-injection easier for patients. A conventional autoinjector may provide the force for administering the injection by a spring, and a trigger button or other mechanism may be used to activate the injection. Autoinjectors may be single-use or reusable devices. Autoinjectors may be single-dose, delivering the entire contents of a cartridge or pre-filled syringe, or may be fixed-dose, delivering predetermined amounts from the cartridge or pre-filled syringe. A reusable autoinjector utilizes disposable safety needles and cartridges or pre-filled syringes. In the latter case, there is a risk of needlestick injury when inserting/removing a pre-filled syringe. Further, most autoinjectors do not verify any use data about the cartridge or syringe prior to, during or after use. Thus, if there is a problem with the cartridge/syringe or its contents, it is up to the patient/physician to determine and address the problem.

There remains a need for a medicament cartridge with a safety mechanism and/or a data storage device.

### Summary of the Invention

It is an object of the present invention to provide a medicament cartridge.

In an exemplary embodiment, a medicament cartridge according to the present invention comprises a housing adapted to contain a syringe containing a medicament, a shield telescopically coupled to the housing and translatable relative to the housing between a retracted position and an extended position, and a data storage device including medicament data.

In an exemplary embodiment, the medicament cartridge further comprises a syringe including a stopper and a needle. The syringe includes a needle sheath removably covering the needle. In the extended position, the shield covers the needle.

In an exemplary embodiment, the medicament cartridge further comprises a lock adapted to lock the shield in the extended position. The lock includes one or more resilient arms adapted to deflect radially when the shield translates from the retracted position to the extended position. When the shield is in the extended position, the one or more arms abut the shield to prevent the shield from returning to the retracted position.

In an exemplary embodiment, the medicament cartridge further comprises a spring biasing the shield relative to the housing.

In an exemplary embodiment, the data storage device includes at least one of read-only memory, random access memory, magnetic memory, optical memory, mechanical memory, a bar code, an RFID tag, a QR code and thermal paper.

In an exemplary embodiment, the medicament cartridge further comprises an actuator in communication with the data storage device and adapted to release the shield from the retracted position.

In an exemplary embodiment, the medicament cartridge further comprises a sensor adapted to generate a signal indicative of at least one of removal of the needle sheath and movement of the shield from the retracted position to the extended position.

In an exemplary embodiment, the medicament data includes at least one of a medicament, a dose, a number of doses provided by the cartridge, a manufacturing date, a batch number, an expiration date, a use indicator, supply chain data, patient data, geographical data, storage condition data, and temperature data.

In an exemplary embodiment, the medicament cartridge further comprises a temperature sensor adapted to generate a signal indicative of a temperature of the medicament.

Further scope of applicability of the present invention will become apparent from the detailed description given hereinafter. However, it should be understood that the detailed description and specific examples, while indicating preferred embodiments of the invention, are given by way of illustration only, since various changes and modifications within the spirit and scope of the invention will become apparent to those skilled in the art from this detailed description.

### Brief Description of the Drawings

The present invention will become more fully understood from the detailed description given hereinbelow and the accompanying drawings which are given by way of illustration only, and thus, are not limitive of the present invention, and wherein:
- Figure 1: shows an exemplary embodiment of a medicament cartridge according to the present invention before use,
- Figure 2: shows an exemplary embodiment of a medicament cartridge according to the present invention during use, and
- Figure 3: shows an exemplary embodiment of a medicament cartridge according to the present invention after use.

Corresponding parts are marked with the same reference symbols in all figures.

### Detailed Description

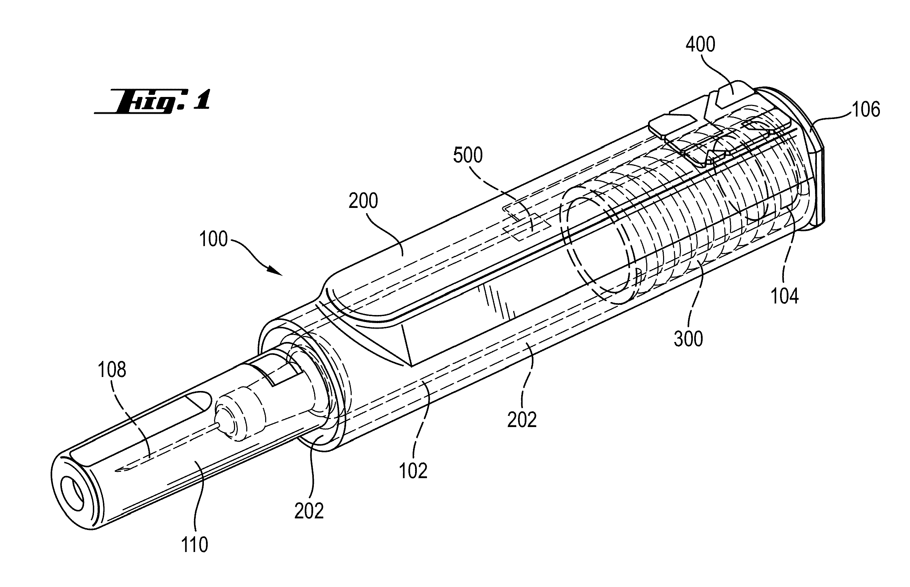

Figure 1 shows an exemplary embodiment of a medicament cartridge 100 according to the present invention. The cartridge 100 is adapted to contain a syringe 102 adapted to contain a medicament. Slidably disposed in the syringe 102 is a stopper 104 which, when translated relative to the syringe 102, causes the medicament to be expelled from the syringe 102.

In an exemplary embodiment, a proximal end of the syringe 102 may include a flange 106 and a distal end of the syringe 102 may include a needle 108. In another exemplary embodiment, the distal end of the syringe 102 may include a coupling (e.g., thread, bayonet coupling, snap fit, friction fit, etc.) adapted to engage a disposable needle assembly. As shown in the exemplary embodiment in Figure 1, the needle 108 may be covered by a needle sheath 110.

In an exemplary embodiment, the syringe 102 is held in a fixed position within a housing 200. The housing 200 may be cylindrical or any other shape and/or size for accommodating the syringe 102. In an exemplary embodiment, the housing 200 may have a particular shape for engaging a carrier in an injection device, e.g., an autoinjector. The particular shape may be useful for compliance, e.g., to ensure that an appropriate cartridge 100 is being used with the injection device. For example, different housing shapes may correspond to different medicaments, different doses, etc.

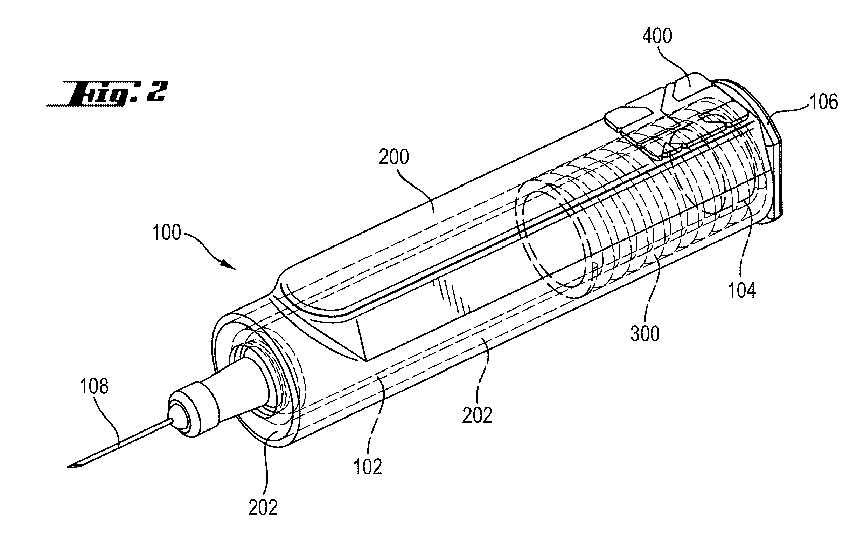

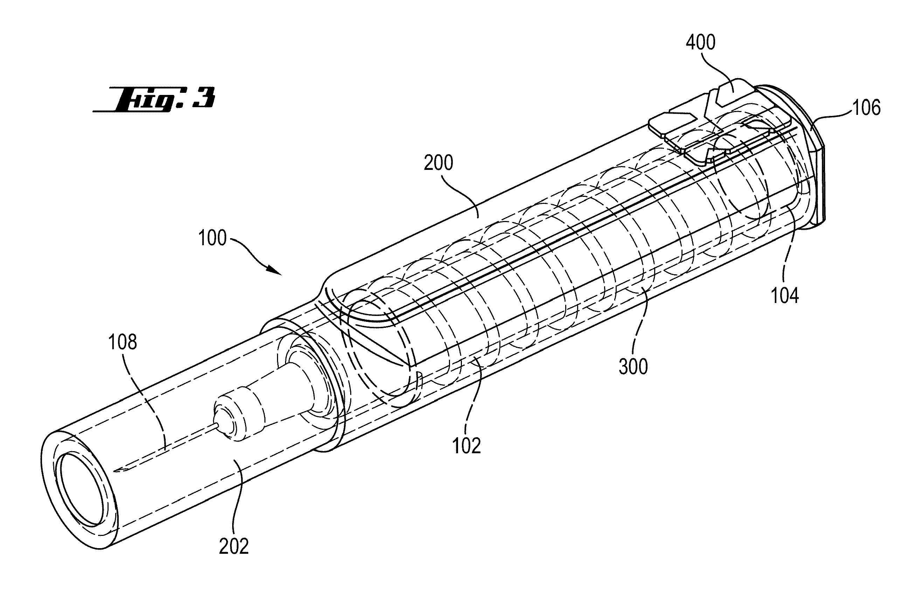

Telescopically coupled to the housing 200 is a shield 202. In an exemplary embodiment, the shield 202 may be contained with the housing 200, and in another exemplary embodiment, the shield 202 may at least partially cover an outer surface of the housing 200. The shield 202 is translatable relative to the housing 200 from a retracted position (as shown in Figures 1 and 2) to an extended position (as shown in Figure 3). In the extended position, the shield 202 may cover the needle 108 to, e.g., prevent needle stick injuries when the cartridge 100 is being removed from the injection device. In an exemplary embodiment, the shield 202 may include a lock which prevents it from returning to the retracted position after it is in the extended position. For example, the lock may include one or more locking resilient locking arms formed on the housing 200 that are deflected radially by the shield 202 as it moves from the retracted position to the extended position. After the shield 202 has reached the extended position, the arms return to a non-deflected position and abut a proximal end of the shield 202 preventing it from returning to the retracted position. Those of skill in the art will understand that other locking mechanisms may be utilized, e.g., friction, snap-fit, etc.

In an exemplary embodiment, a spring 300 may be disposed in the cartridge 100 to bias the shield 202 relative to the housing 200. The spring 300 and/or the shield 202 may be engaged by a retainer to maintain the shield in the retracted position.

In an exemplary embodiment, the cartridge 100 includes a data storage device 400. The data storage device 400 may include, for example, any type of read-only memory (e.g., ROM, EPROM, EEPROM, etc.) and/or any type of random access memory (RAM, SRAM, DRAM, etc.). In another exemplary embodiment, the data storage device 400 may be a radio frequency identification (RFID) tag. In another exemplary embodiment, the data storage device 400 may be magnetic memory, optical memory or mechanical memory.

In an exemplary embodiment, the data storage device 400 may be coupled to an actuator (not shown) which, when activated, releases the spring 300 and/or the shield 202. For example, the actuator may be a pin which engages an abutment surface on the shield 202. When the pin releases the abutment surface (or when the shield 202 rotates relative to the pin), the shield 202 may be releases and moved under the force of the spring 300.

In an exemplary embodiment, the data storage device 400 may be in communication with one or more sensors disposed on the cartridge 100. For example, a first sensor may transmit a signal to the data storage device 400 when the needle sheath 110 is removed. A second sensor may transmit a signal to the data storage device 400 when the spring 300 and/or the shield 202 is released. Those of skill in the art will understand that various other sensors may be disposed on the cartridge 100 for measuring/determining functional parameters of the cartridge 100, housing 200, the shield 202 and the spring 400, and the sensors may utilize any detection mechanism (e.g., optical, mechanical, electrical, magnetic, etc.).

In an exemplary embodiment, the data storage device 400 may store data associated with the cartridge 100. For example, the data may include, but is not limited to, a medicament in the cartridge 100, a dose provided by the cartridge 100, a number of doses provided by the cartridge 100, a manufacturing date of the medicament, a batch number of the medicament, an expiration date of the medicament, a use indicator (whether the cartridge 100 has been used or not), supply chain data (e.g., pharmacist identity), patient data (e.g., encoded with a unique patient's biometric data), geographical data (e.g., this medicament is intended for use in a certain locality), storage condition data, and temperature data (e.g., current temperature of medicament, temperature thresholds of medicament, temperature history of the cartridge and/or the medicament). Those of skill in the art will understand that various other types of data related to the cartridge 100, the medicament, supply chain, regulatory information, patient, physician, insurance, etc.

In another exemplary embodiment, the data storage device 400 may be a printed label, e.g., a bar code, a QR code, thermal paper, etc.

In an exemplary embodiment, the cartridge 100 includes a temperature sensor 500 which senses a temperature of the medicament and/or generates data from which the temperature of the medicament can be determined. The temperature sensor 500 may be in communication with the data storage device 400 and provide temperature data thereto.

In an exemplary embodiment, the cartridge 100 may include a power source (not shown) which provides power to the data storage device 400 and the sensor(s) on the cartridge 100. In another exemplary embodiment, the power for the data storage device 400 and sensor(s) may be provided by an external system (e.g., an interrogator).

The term "drug" or "medicament", as used herein, means a pharmaceutical formulation containing at least one pharmaceutically active compound,
wherein in one embodiment the pharmaceutically active compound has a molecular weight up to 1500 Da and/or is a peptide, a proteine, a polysaccharide, a vaccine, a DNA, a RNA, an enzyme, an antibody or a fragment thereof, a hormone or an oligonucleotide, or a mixture of the above-mentioned pharmaceutically active compound,
wherein in a further embodiment the pharmaceutically active compound is useful for the treatment and/or prophylaxis of diabetes mellitus or complications associated with diabetes mellitus such as diabetic retinopathy, thromboembolism disorders such as deep vein or pulmonary thromboembolism, acute coronary syndrome (ACS), angina, myocardial infarction, cancer, macular degeneration, inflammation, hay fever, atherosclerosis and/or rheumatoid arthritis,
wherein in a further embodiment the pharmaceutically active compound comprises at least one peptide for the treatment and/or prophylaxis of diabetes mellitus or complications associated with diabetes mellitus such as diabetic retinopathy,
wherein in a further embodiment the pharmaceutically active compound comprises at least one human insulin or a human insulin analogue or derivative, glucagon-like peptide (GLP-1) or an analogue or derivative thereof, or exendin-3 or exendin-4 or an analogue or derivative of exendin-3 or exendin-4.

Insulin analogues are for example Gly(A21), Arg(B31), Arg(B32) human insulin; Lys(B3), Glu(B29) human insulin; Lys(B28), Pro(B29) human insulin; Asp(B28) human insulin; human insulin, wherein proline in position B28 is replaced by Asp, Lys, Leu, Val or Ala and wherein in position B29 Lys may be replaced by Pro; Ala(B26) human insulin; Des(B28-B30) human insulin; Des(B27) human insulin and Des(B30) human insulin. Insulin derivates are for example B29-N-myristoyl-des(B30) human insulin; B29-N-palmitoyl-des(B30) human insulin; B29-N-myristoyl human insulin; B29-N-palmitoyl human insulin; B28-N-myristoyl LysB28ProB29 human insulin; B28-N-palmitoyl-LysB28ProB29 human insulin; B30-N-myristoyl-ThrB29LysB30 human insulin; B30-N-palmitoyl- ThrB29LysB30 human insulin; B29-N-(N-palmitoyl-Y-glutamyl)-des(B30) human insulin; B29-N-(N-lithocholyl-Y-glutamyl)-des(B30) human insulin; B29-N-(ω-carboxyheptadecanoyl)-des(B30) human insulin and B29-N-(ω-carboxyheptadecanoyl) human insulin.

Exendin-4 for example means Exendin-4(1-39), a peptide of the sequence H-His-Gly-Glu-Gly-Thr-Phe-Thr-Ser-Asp-Leu-Ser-Lys-Gln-Met-Glu-Glu-Glu-Ala-Val-Arg-Leu-Phe-Ile-Glu-Trp-Leu-Lys-Asn-Gly-Gly-Pro-Ser-Ser-Gly-Ala-Pro-Pro-Pro-Ser-NH2.

Exendin-4 derivatives are for example selected from the following list of compounds:
H-(Lys)4-des Pro36, des Pro37 Exendin-4(1-39)-NH2,
H-(Lys)5-des Pro36, des Pro37 Exendin-4(1-39)-NH2,
des Pro36 Exendin-4(1-39),
des Pro36 [Asp28] Exendin-4(1-39),
des Pro36 [IsoAsp28] Exendin-4(1-39),
des Pro36 [Met(O)14, Asp28] Exendin-4(1-39),
des Pro36 [Met(O)14, IsoAsp28] Exendin-4(1-39),
des Pro36 [Trp(02)25, Asp28] Exendin-4(1-39),
des Pro36 [Trp(02)25, IsoAsp28] Exendin-4(1-39),
des Pro36 [Met(O)14 Trp(02)25, Asp28] Exendin-4(1-39),
des Pro36 [Met(O)14 Trp(02)25, IsoAsp28] Exendin-4(1-39); or
des Pro36 [Asp28] Exendin-4(1-39),
des Pro36 [IsoAsp28] Exendin-4(1-39),
des Pro36 [Met(O)14, Asp28] Exendin-4(1-39),
des Pro36 [Met(O)14, IsoAsp28] Exendin-4(1-39),
des Pro36 [Trp(02)25, Asp28] Exendin-4(1-39),
des Pro36 [Trp(02)25, IsoAsp28] Exendin-4(1-39),
des Pro36 [Met(O)14 Trp(02)25, Asp28] Exendin-4(1-39),
des Pro36 [Met(O)14 Trp(02)25, IsoAsp28] Exendin-4(1-39),
   wherein the group -Lys6-NH2 may be bound to the C-terminus of the Exendin-4 derivative;
   or an Exendin-4 derivative of the sequence
des Pro36 Exendin-4(1-39)-Lys6-NH2 (AVE0010),
H-(Lys)6-des Pro36 [Asp28] Exendin-4(1-39)-Lys6-NH2,
des Asp28 Pro36, Pro37, Pro38Exendin-4(1-39)-NH2,
H-(Lys)6-des Pro36, Pro38 [Asp28] Exendin-4(1-39)-NH2,
H-Asn-(Glu)5des Pro36, Pro37, Pro38 [Asp28] Exendin-4(1-39)-NH2,
des Pro36, Pro37, Pro38 [Asp28] Exendin-4(1-39)-(Lys)6-NH2,
H-(Lys)6-des Pro36, Pro37, Pro38 [Asp28] Exendin-4(1-39)-(Lys)6-NH2,
H-Asn-(Glu)5-des Pro36, Pro37, Pro38 [Asp28] Exendin-4(1-39)-(Lys)6-NH2,
H-(Lys)6-des Pro36 [Trp(02)25, Asp28] Exendin-4(1-39)-Lys6-NH2,
H-des Asp28 Pro36, Pro37, Pro38 [Trp(02)25] Exendin-4(1-39)-NH2,
H-(Lys)6-des Pro36, Pro37, Pro38 [Trp(02)25, Asp28] Exendin-4(1-39)-NH2,
H-Asn-(Glu)5-des Pro36, Pro37, Pro38 [Trp(02)25, Asp28] Exendin-4(1-39)-NH2,
des Pro36, Pro37, Pro38 [Trp(02)25, Asp28] Exendin-4(1-39)-(Lys)6-NH2,
H-(Lys)6-des Pro36, Pro37, Pro38 [Trp(02)25, Asp28] Exendin-4(1-39)-(Lys)6-NH2,
H-Asn-(Glu)5-des Pro36, Pro37, Pro38 [Trp(02)25, Asp28] Exendin-4(1-39)-(Lys)6-NH2,
H-(Lys)6-des Pro36 [Met(O)14, Asp28] Exendin-4(1-39)-Lys6-NH2,
des Met(O)14 Asp28 Pro36, Pro37, Pro38 Exendin-4(1-39)-NH2,
H-(Lys)6-desPro36, Pro37, Pro38 [Met(O)14, Asp28] Exendin-4(1-39)-NH2,
H-Asn-(Glu)5-des Pro36, Pro37, Pro38 [Met(O)14, Asp28] Exendin-4(1-39)-NH2,
des Pro36, Pro37, Pro38 [Met(O)14, Asp28] Exendin-4(1-39)-(Lys)6-NH2,
H-(Lys)6-des Pro36, Pro37, Pro38 [Met(O)14, Asp28] Exendin-4(1-39)-(Lys)6-NH2,
H-Asn-(Glu)5 des Pro36, Pro37, Pro38 [Met(O)14, Asp28] Exendin-4(1-39)-(Lys)6-NH2,
H-Lys6-des Pro36 [Met(O)14, Trp(02)25, Asp28] Exendin-4(1-39)-Lys6-NH2,
H-des Asp28 Pro36, Pro37, Pro38 [Met(O)14, Trp(02)25] Exendin-4(1-39)-NH2,
H-(Lys)6-des Pro36, Pro37, Pro38 [Met(O)14, Asp28] Exendin-4(1-39)-NH2,
H-Asn-(Glu)5-des Pro36, Pro37, Pro38 [Met(O)14, Trp(02)25, Asp28] Exendin-4(1-39)-NH2,
des Pro36, Pro37, Pro38 [Met(O)14, Trp(02)25, Asp28] Exendin-4(1-39)-(Lys)6-NH2,
H-(Lys)6-des Pro36, Pro37, Pro38 [Met(O)14, Trp(02)25, Asp28] Exendin-4(S1-39)-(Lys)6-NH2,
H-Asn-(Glu)5-des Pro36, Pro37, Pro38 [Met(O)14, Trp(02)25, Asp28] Exendin-4(1-39)-(Lys)6-NH2;
   or a pharmaceutically acceptable salt or solvate of any one of the afore-mentioned Exendin-4 derivative.

Hormones are for example hypophysis hormones or hypothalamus hormones or regulatory active peptides and their antagonists as listed in Rote Liste, ed. 2008, Chapter 50, such as Gonadotropine (Follitropin, Lutropin, Choriongonadotropin, Menotropin), Somatropine (Somatropin), Desmopressin, Terlipressin, Gonadorelin, Triptorelin, Leuprorelin, Buserelin, Nafarelin, Goserelin.

A polysaccharide is for example a glucosaminoglycane, a hyaluronic acid, a heparin, a low molecular weight heparin or an ultra low molecular weight heparin or a derivative thereof, or a sulphated, e.g. a poly-sulphated form of the above-mentioned polysaccharides, and/or a pharmaceutically acceptable salt thereof. An example of a pharmaceutically acceptable salt of a poly-sulphated low molecular weight heparin is enoxaparin sodium.

Antibodies are globular plasma proteins (∼150 kDa) that are also known as immunoglobulins which share a basic structure. As they have sugar chains added to amino acid residues, they are glycoproteins. The basic functional unit of each antibody is an immunoglobulin (Ig) monomer (containing only one Ig unit); secreted antibodies can also be dimeric with two Ig units as with IgA, tetrameric with four Ig units like teleost fish IgM, or pentameric with five Ig units, like mammalian IgM.

The Ig monomer is a "Y"-shaped molecule that consists of four polypeptide chains; two identical heavy chains and two identical light chains connected by disulfide bonds between cysteine residues. Each heavy chain is about 440 amino acids long; each light chain is about 220 amino acids long. Heavy and light chains each contain intrachain disulfide bonds which stabilize their folding. Each chain is composed of structural domains called Ig domains. These domains contain about 70-110 amino acids and are classified into different categories (for example, variable or V, and constant or C) according to their size and function. They have a characteristic immunoglobulin fold in which two β sheets create a "sandwich" shape, held together by interactions between conserved cysteines and other charged amino acids.

There are five types of mammalian Ig heavy chain denoted by α, δ, ε, γ, and µ. The type of heavy chain present defines the isotype of antibody; these chains are found in IgA, IgD, IgE, IgG, and IgM antibodies, respectively.

Distinct heavy chains differ in size and composition; α and γ contain approximately 450 amino acids and δ approximately 500 amino acids, while µ and ε have approximately 550 amino acids. Each heavy chain has two regions, the constant region (C_{H}) and the variable region (V_{H}). In one species, the constant region is essentially identical in all antibodies of the same isotype, but differs in antibodies of different isotypes. Heavy chains γ, α and δ have a constant region composed of three tandem Ig domains, and a hinge region for added flexibility; heavy chains µ and ε have a constant region composed of four immunoglobulin domains. The variable region of the heavy chain differs in antibodies produced by different B cells, but is the same for all antibodies produced by a single B cell or B cell clone. The variable region of each heavy chain is approximately 110 amino acids long and is composed of a single Ig domain.

In mammals, there are two types of immunoglobulin light chain denoted by λ and κ. A light chain has two successive domains: one constant domain (CL) and one variable domain (VL). The approximate length of a light chain is 211 to 217 amino acids. Each antibody contains two light chains that are always identical; only one type of light chain, κ or λ, is present per antibody in mammals.

Although the general structure of all antibodies is very similar, the unique property of a given antibody is determined by the variable (V) regions, as detailed above. More specifically, variable loops, three each the light (VL) and three on the heavy (VH) chain, are responsible for binding to the antigen, i.e. for its antigen specificity. These loops are referred to as the Complementarity Determining Regions (CDRs). Because CDRs from both VH and VL domains contribute to the antigen-binding site, it is the combination of the heavy and the light chains, and not either alone, that determines the final antigen specificity.

An "antibody fragment" contains at least one antigen binding fragment as defined above, and exhibits essentially the same function and specificity as the complete antibody of which the fragment is derived from. Limited proteolytic digestion with papain cleaves the Ig prototype into three fragments. Two identical amino terminal fragments, each containing one entire L chain and about half an H chain, are the antigen binding fragments (Fab). The third fragment, similar in size but containing the carboxyl terminal half of both heavy chains with their interchain disulfide bond, is the crystalizable fragment (Fc). The Fc contains carbohydrates, complement-binding, and FcR-binding sites. Limited pepsin digestion yields a single F(ab')2 fragment containing both Fab pieces and the hinge region, including the H-H interchain disulfide bond. F(ab')2 is divalent for antigen binding. The disulfide bond of F(ab')2 may be cleaved in order to obtain Fab'. Moreover, the variable regions of the heavy and light chains can be fused together to form a single chain variable fragment (scFv).

Pharmaceutically acceptable salts are for example acid addition salts and basic salts. Acid addition salts are e.g. HCl or HBr salts. Basic salts are e.g. salts having a cation selected from alkali or alkaline, e.g. Na+, or K+, or Ca2+, or an ammonium ion N+(R1)(R2)(R3)(R4), wherein R1 to R4 independently of each other mean: hydrogen, an optionally substituted C1-C6-alkyl group, an optionally substituted C2-C6-alkenyl group, an optionally substituted C6-C10-aryl group, or an optionally substituted C6-C10-heteroaryl group. Further examples of pharmaceutically acceptable salts are described in "Remington's Pharmaceutical Sciences" 17. ed. Alfonso R. Gennaro (Ed.), Mark Publishing Company, Easton, Pa., U.S.A., 1985 and in Encyclopedia of Pharmaceutical Technology.

Pharmaceutically acceptable solvates are for example hydrates.

Those of skill in the art will understand that modifications (additions and/or removals) of various components of the apparatuses, methods and/or systems and embodiments described herein may be made without departing from the full scope and spirit of the present invention, which encompass such modifications and any and all equivalents thereof.

## Claims

1. A medicament cartridge comprising:
a housing (200) adapted to contain a syringe (102) containing a medicament;
a shield (202) telescopically coupled to the housing (200) and translatable relative to the housing (200) between a retracted position and an extended position; and
a data storage device (400) including medicament data.

2. The medicament cartridge according to claim 1, further comprising:
a syringe (102) including a stopper (104) and a needle (108).

3. The medicament cartridge according to claim 2, wherein the syringe (102) includes a needle sheath (110) removably covering the needle (108).

4. The medicament cartridge according to claims 2 or 3, wherein, in the extended position, the shield (202) covers the needle (108).

5. The medicament cartridge according to any one of the preceding claims, further comprising:
a lock adapted to lock the shield in the extended position.

6. The medicament cartridge according to claims 5, wherein the lock includes one or more resilient arms adapted to deflect radially when the shield (202) translates from the retracted position to the extended position, wherein when the shield (202) is in the extended position, the one or more arms abut the shield (202) to prevent the shield (202) from returning to the retracted position.

7. The medicament cartridge according to any one of the preceding claims, further comprising:
a spring (300) biasing the shield (202) relative to the housing (200).

8. The medicament cartridge according to any one of the preceding claims, wherein the data storage device (400) includes at least one of read-only memory, random access memory, magnetic memory, optical memory, mechanical memory, a bar code, an RFID tag, a QR code and thermal paper.

9. The medicament cartridge according to any one of the preceding claims, further comprising:
an actuator in communication with the data storage device (400) and adapted to release the shield (202) from the retracted position.

10. The medicament cartridge according to claim 3, further comprising:
a sensor adapted to generate a signal indicative of at least one of removal of the needle sheath (110) and movement of the shield (202) from the retracted position to the extended position.

11. The medicament cartridge according to any one of the preceding claims, wherein the medicament data includes at least one of a medicament, a dose, a number of doses provided by the cartridge (100), a manufacturing date, a batch number, an expiration date, a use indicator, supply chain data, patient data, geographical data, storage condition data, and temperature data.

12. The medicament cartridge according to any one of the preceding claims, further comprising:
a temperature sensor (500) adapted to generate a signal indicative of a temperature of the medicament.
